# EUROPEAN PATENT APPLICATION

(11) **EP 4 628 085 A1**
(43) Date of publication of application: **08.10.2025**
(21) Application number: 23897707.8
(22) Date of filing: 27.11.2023
(51) Int. Cl.: A61K 35/16, A61K 9/06, A61K 9/70, A61K 47/02, A61K 47/10, A61K 47/42, A61P 17/02, A61K 35/19

(54) **METHOD FOR PRODUCING GEL-LIKE WOUND HEALING AGENT, GEL-LIKE WOUND HEALING AGENT, AND AUTOLOGOUS PLATELET-RICH PLASMA GEL PREPARATION KIT FOR WOUND HEALING**

(30) Priority: 02.12.2022 JP 2022193847
(71) Applicant: Rohto Pharmaceutical Co., Ltd., Osaka-shi, Osaka 544-8666 (JP)
(72) Inventor: SHOJU Shintaro, Osaka-shi, Osaka 544-8666 (JP); KAWAI Mami, Osaka-shi, Osaka 544-8666 (JP); MITSUGUCHI Yoko, Osaka-shi, Osaka 544-8666 (JP)
(74) Representative: Mewburn Ellis LLP
(86) International application number: PCT/JP2023/042289
(87) International publication number: WO 2024/117059

(57) **Abstract**

The purpose of the present invention is to provide a treatment method and therapeutic agent by which a sufficient effect can be obtained even for a wound or the like for which no tendency toward improvement is observed with existing treatment methods. The present invention pertains to a method for producing a gel-like wound healing agent, the method comprising a feature of mixing an effective amount of a platelet-rich plasma (A), 150-300 mg/mL of an ascorbic acid solution (B), 1.0-3.0 wt% of a calcium chloride solution (C), and thrombin (D), a mixing ratio of the platelet-rich plasma (A) and the ascorbic acid solution (B) being 1.0-3.0 mL of the ascorbic acid solution (B) per 8.0 mL of the platelet-rich plasma (A), a mixing ratio of the platelet-rich plasma (A) and the calcium chloride solution (C) being 1.0-3.0 mL of the calcium chloride solution (C) per 8.0 mL of the platelet-rich plasma (A), and the amount of thrombin (D) being 500-2000 U per 8.0 mL of the platelet-rich plasma (A).

## Description

### TECHNICAL FIELD

The present invention relates to a method for producing a wound healing gel, a wound healing gel, and an autologous platelet-rich plasma gel preparation kit for wound healing.

### BACKGROUND ART

In Japan, it is estimated that there are 10 million diabetes patients, and more than 20 million including those at risk. One of the complications of diabetes is diabetic ulcers, and some reports indicate that the prevalence of skin ulcers in diabetic patients can be as high as about 15%. With the aging population, the number of diabetes patients is on the rise, and an increase of patients with diabetic ulcers, a complication of diabetes, is also expected. Severe diabetic ulcers can lead to amputation of lower limb, so it is considered important to actively treat the wounds of diabetic ulcers and prevent the deterioration of the condition from an early stage. The usefulness of autologous platelet-rich plasma (PRP) therapy for such wound treatment has been reported overseas (see Patent Literatures 1 and 2).

Autologous platelet-rich plasma (PRP) therapy is a treatment method which promotes tissue regeneration and leads to the healing of wound site by administering concentrated platelet-rich plasma, separated from blood taken from the patient, to damaged tissues, and thus utilizing the effects of various highly concentrated growth factors contained in the platelet-rich plasma. Differences in the amounts of platelets (platelet concentration rates) in platelet-rich plasma, variations in the processing methods of platelet-rich plasma, and the like bring about different therapeutic effects obtained.

There is a need for therapies and therapeutic agents which can improve the issues associated with the conventionally known autologous platelet-rich plasma (PRP) therapies and can provide sufficient effects for wounds and the like which do not show improvement trends with existing therapies.

### CITATION LIST

### Patent Literature

| | |
|---|---|
| Patent Literature 1 | JP 2003-524590 A |
| Patent Literature 2 | JP 2013-507225 A |

### SUMMARY OF INVENTION

### Technical Problem

The present invention has been made based on the circumstances described above, and aims to provide a therapy and a therapeutic agent which can achieve sufficient effects even for wounds and the like which do not show improvement trends with existing therapies.

### Solution to Problem

**In** order to solve above problem, the present inventors conducted intensive studies and found it possible to produce a wound healing gel using autologous platelet-rich plasma (PRP), by applying a specific combination of concentrations for ascorbic acid and calcium chloride to be mixed during the production, which achieves appropriate hardness as wound healing gel and can maintain its form for a long time to sufficiently protect the wound site when applied to skin wounds, thus enabling to achieve a high therapeutic effect. Furthermore, the wound healing gel was confirmed to achieve sufficient effects on the wounds and the like, which have not shown improvement trends with existing therapies. The present invention has been completed based on these findings.

That is, the summary of the present invention is as follows.
[1] A method for producing a wound healing gel, comprising mixing:
   (A) an effective amount of platelet-rich plasma,
   (B) an ascorbic acid solution at a concentration of 150 to 300 mg/mL,
   (C) a calcium chloride solution at a concentration of 1.0 to 3.0 wt.%, and
   (D) thrombin,
   wherein
   a mixing ratio between (A) platelet-rich plasma and (B) ascorbic acid solution is 1.0 to 3.0 mL of (B) ascorbic acid solution per 8.0 mL of (A) platelet-rich plasma,
   a mixing ratio between (A) platelet-rich plasma and (C) calcium chloride solution is 1.0 to 3.0 mL of (C) calcium chloride solution per 8.0 mL of (A) platelet-rich plasma, and
   an amount of (D) thrombin is 500 to 2,000 U per 8.0 mL of (A) platelet-rich plasma.
[2] The method for producing a wound healing gel according to [1], wherein (D) thrombin being dissolved in (C) calcium chloride solution is mixed with (A) platelet-rich plasma and (B) ascorbic acid solution.
[3] The method for producing a wound healing gel according to [2], wherein with (A) platelet-rich plasma, (B) ascorbic acid solution is mixed, and then the calcium chloride solution of thrombin is mixed.
[4] The method for producing a wound healing gel according to [1], further comprising obtaining (A) platelet-rich plasma by centrifuging blood to separate platelet concentrates from other blood components.
[5] The method for producing a wound healing gel according to [4], wherein the blood is treated with a calcium chelator.
[6] The method for producing a wound healing gel according to [4], wherein the conditions of the centrifugation is 3,000 to 4,500 ×*g* for 20 seconds to 1 minute.
[7] A method for producing a wound healing gel sheet, comprising mixing:
   (A) an effective amount of platelet-rich plasma,
   (B) an ascorbic acid solution at a concentration of 150 to 300 mg/mL,
   (C) a calcium chloride solution at a concentration of 1.0 to 3.0 wt.%, and
   (D) thrombin,
   wherein
   a mixing ratio between (A) platelet-rich plasma and (B) ascorbic acid solution is 1.0 to 3.0 mL of (B) ascorbic acid solution per 8.0 mL of (A) platelet-rich plasma,
   a mixing ratio between (A) platelet-rich plasma and (C) calcium chloride solution is 1.0 to 3.0 mL of (C) calcium chloride solution per 8.0 mL of (A) platelet-rich plasma, and
   an amount of (D) thrombin is 500 to 2,000 U per 8.0 mL of (A) platelet-rich plasma.
[8] A wound healing gel obtained by mixing:
   (A) an effective amount of platelet-rich plasma,
   (B) an ascorbic acid solution at a concentration of 150 to 300 mg/mL,
   (C) a calcium chloride solution at a concentration of 1.0 to 3.0 wt.%, and
   (D) thrombin,
   wherein
   a mixing ratio between (A) platelet-rich plasma and (B) ascorbic acid solution is 1.0 to 3.0 mL of (B) ascorbic acid solution per 8.0 mL of (A) platelet-rich plasma, and
   a mixing ratio between (A) platelet-rich plasma and (C) calcium chloride solution is 1.0 to 3.0 mL of (C) calcium chloride solution per 8.0 mL of (A) platelet-rich plasma.
[9] The wound healing gel according to [8], wherein (D) thrombin being dissolved in (C) calcium chloride solution is mixed with (A) platelet-rich plasma and (B) ascorbic acid solution.
[10] The wound healing gel according to [9], wherein the wound healing gel is obtained by mixing with (A) platelet-rich plasma, (B) ascorbic acid solution, and then the calcium chloride solution of thrombin.
[11] An autologous platelet-rich plasma gel preparation kit for wound healing, consisting of:
   (I) blood collection needle,
   (II) blood collection tube, and
   (III) gelation reagent set,
   wherein (III) gelation reagent set contains ascorbic acid solution at a concentration of 150 to 300 mg/mL, calcium chloride solution at a concentration of 1.0 to 3.0 wt.%, and thrombin in separate forms.

### Advantageous Effects of Invention

The wound healing gel produced by the method of the present invention is firmly gelled compared to conventionally known wound healing gels using platelet-rich plasma, enabling to maintain its form for a long time when applied to skin wounds, sufficiently protecting the wound site and achieving a high therapeutic effect, also achieving sufficient effects on wounds and the like, which have not shown improvement trends with existing therapies. Additionally, it has the advantage of being easy to handle because it is firmly gelled.

### BRIEF DESCRIPTION OF DRAWINGS

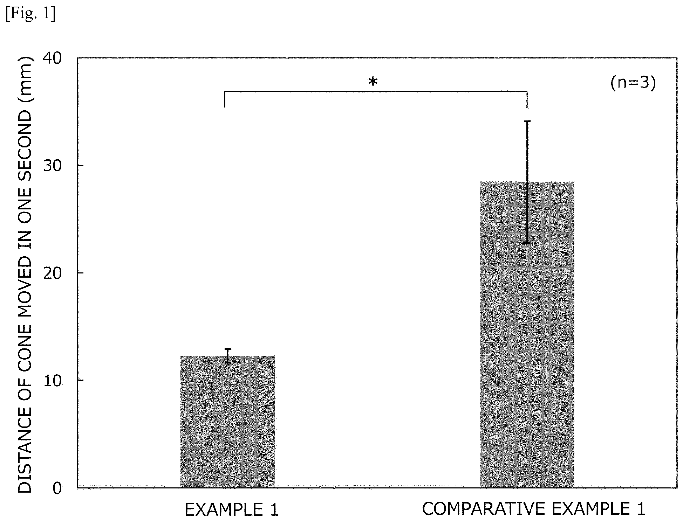

[Fig. 1] Fig. 1 shows a comparison of the hardness of the wound healing gel of the present invention and a conventional product.

### DESCRIPTION OF EMBODIMENTS

Hereinafter, the method for producing the wound healing gel, the wound healing gel, and the autologous platelet-rich plasma gel preparation kit for wound healing of the present invention are described in detail.

### <Method for producing the wound healing gel>

The method for producing a wound healing gel of the present invention comprises mixing (A) an effective amount of platelet-rich plasma, (B) an ascorbic acid solution at a concentration of 150 to 300 mg/mL, (C) a calcium chloride solution at a concentration of 1.0 to 3.0 wt.%, and (D) thrombin,
wherein
a mixing ratio between (A) platelet-rich plasma and (B) ascorbic acid solution is 1.0 to 3.0 mL of (B) ascorbic acid solution per 8.0 mL of (A) platelet-rich plasma,
a mixing ratio between (A) platelet-rich plasma and (C) calcium chloride solution is 1.0 to 3.0 mL of (C) calcium chloride solution per 8.0 mL of (A) platelet-rich plasma, and
an amount of (D) thrombin is 500 to 2,000 U per 8.0 mL of (A) platelet-rich plasma.

The method for producing a wound healing gel of the present invention can further comprise a step of obtaining (A) platelet-rich plasma by centrifuging blood to separate platelet concentrates from other blood components before the step of mixing (A) an effective amount of platelet-rich plasma, (B) an ascorbic acid solution at a concentration of 150 to 300 mg/mL, (C) a calcium chloride solution at a concentration of 1.0 to 3.0 wt.%, and (D) thrombin.

The wound healing gel produced by the method of the present invention is firmly gelled compared to conventionally known wound healing gels using platelet-rich plasma, enabling to maintain its form for a long time when applied to skin wounds, sufficiently protecting the wound site and achieving a high therapeutic effect, also achieving sufficient effects on wounds and the like, which have not shown improvement trends with existing therapies. Additionally, it has the advantage of being easy to handle because it is firmly gelled.

Hereinafter, each requirement constituting the present invention is specifically described.

### [(A) Effective amount of platelet-rich plasma]

The platelet-rich plasma used in the method for producing the wound healing gel of the present invention can be obtained by separating it from red blood cells and white blood cells using such methods as centrifugation of whole blood. The platelet concentration (platelet concentration rate) of the platelet-rich plasma to obtain can be adjusted by the conditions of centrifugation. The platelet-rich plasma used in the method for producing the wound healing gel of the present invention is preferably at a physiological platelet concentration (platelet concentration rate: 1.0 times). The conditions for centrifugation to obtain platelet-rich plasma at a physiological platelet concentration (platelet concentration rate: 1.0 times) are 2,000 to 5,000 ×*g* for 15 seconds to 3 minutes, preferably 3,000 to 4,500 ×*g* for 20 seconds to 1 minute, more preferably 4,000 to 4,500 ×*g* for 20 seconds to 45 seconds, even more preferably 4,000 to 4,500 ×*g* for 30 seconds, and particularly preferably at 4,236 ×*g* for 30 seconds. It is also known that when the platelet concentration in platelet-rich plasma becomes high, it negatively affects cells proliferation at the wound site and cells migration for tissue regeneration (Reese RJ. Autologous platelet rich plasma (PRP): what do we know? Important concepts relevant to hair restoration surgery. Hair Transplant Forum Int 2010:14-7). In the present invention, the wound healing effect of the wound healing gel can be improved by setting the platelet concentration of the platelet-rich plasma at a physiological concentration.

During blood collection, it is necessary to add anticoagulants to prevent blood coagulation. Calcium chelators are preferred as anticoagulants. Examples of calcium chelators which can be used in the present invention include hydroxycarboxylic acid chelators, aminopolycarboxylic acid chelators, aromatic or aliphatic carboxylic acid chelators, amino acid chelators, ether carboxylic acid chelators, phosphonic acid chelators, polymer electrolytes (including oligomer electrolytes) chelators, polyalcohols, nitrogen-containing chelators such as dimethylglyoxime, and sulfur-containing chelators such as thioglycolic acid, among which hydroxycarboxylic acid chelators are preferred.

Examples of hydroxycarboxylic acid chelators include malic acid, citric acid, glycolic acid, gluconic acid, heptanoic acid, tartaric acid, lactic acid, and salts and derivatives thereof. The commercially available products include ACD-A solution (Biological Product Standard Blood Preservation Solution A), which is preferably added in an amount to achieve the final concentration of about 10% in whole blood.

The blood collection can be performed using known methods in its technical field, and for blood collection, materials such as blood collection needles, blood collection tubes, and blood collection bags, as well as equipment, devices and the like can be used.

The platelet-rich plasma of the present invention can also contain blood cells components other than platelets, such as red blood cells and white blood cells.

The platelet-rich plasma of the present invention can be autologous, obtained from the wound patient's own whole blood, or can be derived from whole blood obtained from one or more third parties. Furthermore, as far as biological incompatibility does not occur, the third parties do not have to be of the same species as the wound patient. Among these options, from the perspectives of biological compatibility, safety, and the like, it is preferable that the platelet-rich plasma is autologous, obtained from the wound patient's own whole blood.

It is considered that platelets contained in the platelet-rich plasma of the present invention, when activated by thrombin, collagen, serotonin, adenosine diphosphate (ADP), acetylcholine (ACh) and the like, release alpha granules and the like containing growth factors such as PDGF, coagulation factors, and the like (as explained in the paragraph below) and promote tissue regeneration and the like at the wound site.

Platelets contained in the platelet-rich plasma of the present invention contain the factors selected from the group consisting of platelet-derived growth factor (PDGF), platelet-derived angiogenesis factor (PDAF), vascular endothelial growth factor (VEGF), platelet-derived epithelial growth factor (PDEGF), platelet factor 4 (PF-4), transforming growth factor beta (TGF-β), acidic fibroblast growth factor (FGF-A), basic fibroblast growth factor (FGF-B), transforming growth factor alpha (TGF-α), insulin-like growth factors 1 and 2 (IGF-1 and IGF-2), beta-thromboglobulin-related protein (BTG), thrombospondin (TSP), fibronectin, von Willebrand factor (VWF), fibrinopeptide A, fibrinogen, albumin, plasminogen activator inhibitor 1 (PAI-1), osteonectin, RANTES (regulated upon activation normal T cells expressed and presumably secreted), gro-α, vitronectin, fibrin D dimer, factor V, antithrombin III, immunoglobulin G (IgG), immunoglobulin M (IgM), immunoglobulin A (IgA), alpha-2-macroglobulin, angiogenin, Fg-D, elastase, keratinocyte growth factor (KGF), epidermal growth factor (EGF), fibroblast growth factor (FGF), tumor necrosis factor (TNF), fibroblast growth factor (FGF), and interleukin-1 (IL-1). As one of the common important characteristics of the factors, it is known or considered that each factor enhances the proliferation of cells or tissues and additionally that those factors or various combinations thereof can function synergistically in unexpected ways to promote wound healing.

In the context of "effective amount of platelet-rich plasma," the effective amount refers to the quantity necessary for wound healing (for example, reduction in wound volume or surface area; increase in collagen adhesion, angiogenesis, fibroblast proliferation, or overall healing promoted by the increase of granulation tissue and other biological substances). Furthermore, all the embodiments described herein include an effective amount of the component substances or combinations thereof for wound treatment.

### [(B) Ascorbic acid solution]

In the method for producing a wound healing gel of the present invention, (A) effective amount of platelet-rich plasma described above is mixed with an ascorbic acid solution at a concentration of 150 to 300 mg/mL. The mixing ratio between (A) platelet-rich plasma and (B) ascorbic acid solution is such that per 8.0 mL of (A) platelet-rich plasma, there is at least 1.0 mL or more of (B) ascorbic acid solution, preferably 1.0 to 3.0 mL of (B) ascorbic acid solution, more preferably 1.0 to 2.0 mL of (B) ascorbic acid solution, and even more preferably 1.0 mL of (B) ascorbic acid solution. The concentration of ascorbic acid solution is 150 to 300 mg/mL, preferably 200 to 300 mg/mL, more preferably 220 to 270 mg/mL, and even more preferably 250 mg/mL. By maintaining the mixing ratio between (A) platelet-rich plasma and (B) ascorbic acid solution and the concentration of the ascorbic acid solution within above ranges, it becomes possible to keep the hardness and viscosity of the wound healing gel at appropriate levels. Here, the terms "hardness" and "viscosity" refer to the characteristics used to assess the gelation level of wound healing gels, indicating the degree of firmness, hardness, softness, and fluidity of the wound healing gels.

### [(C) Calcium chloride solution]

In the method for producing a wound healing gel of the present invention, after mixing the (A) effective amount of platelet-rich plasma described above with an ascorbic acid solution at a concentration of 150 to 300 mg/mL, a calcium chloride solution at a concentration of 1.0 to 3.0 wt.% is further mixed in. By mixing in (C) calcium chloride solution, it becomes possible to neutralize the effect of the calcium chelator added during blood collection, enabling the progression of calcium-dependent enzymatic reactions. The concentration of calcium chloride solution is preferably 1.0 to 3.0 wt.%, more preferably 1.5 to 3.0 wt.%, even more preferably 1.5 to 2.5 wt.%, and particularly preferably 2.0 wt.%. As for amount of (C) calcium chloride solution, the mixing ratio between (A) platelet-rich plasma and (C) calcium chloride solution is such that per 8.0 mL of (A) platelet-rich plasma, there is at least 1.0 mL or more of (C) calcium chloride solution, preferably 1.0 to 3.0 mL of (C) calcium chloride solution, more preferably 1.0 to 2.0 mL of (C) calcium chloride solution, and even more preferably 1.0 mL of (C) calcium chloride solution. By maintaining the mixing ratio between (A) platelet-rich plasma and (C) calcium chloride solution and the concentration of the calcium chloride solution within above ranges, it becomes possible to keep the hardness and viscosity of the wound healing gel at appropriate levels in combination with the (B) ascorbic acid solution described above.

### [(D) Thrombin]

In the present invention, thrombin refers to an endoprotease involved in blood coagulation which converts fibrinogen into fibrin, and in humans for example, it specifically refers to the substance known as activated factor II.

(D) thrombin used in the present invention can be obtained by subjecting prothrombin separated from blood or bone marrow by conventional methods to activated factor X and/or calcium ions, phospholipids, and activated factor V. Or, recombinant thrombin can also be used. The origin of the thrombin used in the present invention is not particularly limited, and for example, thrombins derived from bovine blood are widely used.

In the method for producing a wound healing gel of the present invention, (D) thrombin of amount of 500 to 2,000 U is mixed per 8.0 mL of (A) platelet-rich plasma. The amount of (D) thrombin per 8.0 mL of (A) platelet-rich plasma is preferably 500 to 1,500 U, more preferably 750 to 1,250 U, and even more preferably about 1,000 U. Furthermore, since the mixing of thrombin activates platelets and promotes aggregation, it is necessary to mix thrombin just before the wound healing gel of the present invention is desired to be in gel-like form.

In the method for producing a wound healing gel of the present invention, it is preferable that (D) thrombin being dissolved in (C) calcium chloride solution is mixed with (A) platelet-rich plasma and (B) ascorbic acid solution. To prevent the inactivation of (D) thrombin, it is preferable to dissolve it in (C) calcium chloride solution during preparation.

### [Preparation procedure for wound healing gel]

Hereinafter, a preparation procedure for wound healing gel of the present invention is specifically described. Furthermore, the present invention is not limited to the specific descriptions mentioned below.
(1) Collect 4.5 mL of blood from the patients themselves or a third party. During blood collection, add 0.5 mL of anticoagulant such as calcium chelator to prevent blood coagulation.
(2) Centrifuge the blood with anticoagulants added under the conditions described above to obtain (A) platelet-rich plasma at a physiological platelet concentration (platelet concentration ratio: 1.0 times).
(3) Mix 2.5 mL of (A) platelet-rich plasma with (B) ascorbic acid solution of an amount approximately equivalent to one-eighth of the amount of the plasma.
(4) To the mixed solution obtained in (3), add and mix (C) calcium chloride solution dissolved with (D) thrombin of equal amount with that of (B) ascorbic acid solution, and gelate to produce the wound healing gel of the present invention.

The prepared wound healing gel is used for treatment immediately after preparation.

### <Preparation procedure for wound healing gel sheet >

The wound healing gel obtained by the method of the present invention can be formed into a sheet, so the present invention comprises a method for producing a wound healing gel sheet, which comprises mixing (A) an effective amount of platelet-rich plasma, (B) an ascorbic acid solution at a concentration of 150 to 300 mg/mL, (C) a calcium chloride solution at a concentration of 1.0 to 3.0 wt.%, and (D) thrombin,
wherein
a mixing ratio between (A) platelet-rich plasma and (B) ascorbic acid solution is 1.0 to 3.0 mL of (B) ascorbic acid solution per 8.0 mL of (A) platelet-rich plasma,
a mixing ratio between (A) platelet-rich plasma and (C) calcium chloride solution is 1.0 to 3.0 mL of (C) calcium chloride solution per 8.0 mL of (A) platelet-rich plasma, and
an amount of (D) thrombin is 500 to 2,000 U per 8.0 mL of (A) platelet-rich plasma.

Above description of the method for producing wound healing gel can be directly applied to the specific description of the method for producing the wound healing gel sheet of the present invention.

### <Wound healing gel>

The wound healing gel of the present invention is obtained by mixing (A) an effective amount of platelet-rich plasma, (B) an ascorbic acid solution at a concentration of 150 to 300 mg/mL, (C) a calcium chloride solution at a concentration of 1.0 to 3.0 wt.%, and (D) thrombin, wherein a mixing ratio between (A) platelet-rich plasma and (B) ascorbic acid solution is 1.0 to 3.0 mL of (B) ascorbic acid solution per 8.0 mL of (A) platelet-rich plasma, and a mixing ratio between (A) platelet-rich plasma and (C) calcium chloride solution is 1.0 to 3.0 mL of (C) calcium chloride solution per 8.0 mL of (A) platelet-rich plasma. Here, it is preferable that (D) thrombin being dissolved in (C) calcium chloride solution is mixed with (A) platelet-rich plasma and (B) ascorbic acid solution. Also, the wound healing gel of the present invention is preferably obtained by mixing with (A) platelet-rich plasma, (B) ascorbic acid solution, and then the above calcium chloride solution of thrombin.

The wound healing gel of the present invention is produced by the method described above for producing a wound healing gel of the present invention. The wound healing gel of the present invention, by applying above combination of concentrations for ascorbic acid and calcium chloride when mixing during the production, can achieve appropriate hardness as wound healing gel and can maintain its form for a long time to sufficiently protect the wound site when applied to skin wounds, thus enabling to achieve a high therapeutic effect. Furthermore, the wound healing gel can achieve sufficient effects on wounds and the like, which have not shown improvement trends with existing therapies. Additionally, it has the advantage of being easy to handle, because it has appropriate hardness as wound healing gel and it is in the form of sheet.

The types of wounds for which the wound healing gel of the present invention can be used include, but are not particularly limited to, chronic wounds, wounds due to obesity or diabetes (non-healing diabetic wounds, diabetic ulcers, diabetic neuropathic ulcers, other general diabetic wounds, etc.), burns, pressure sores, wounds due to radiation damage, wounds caused by infection, wounds due to ischemia, neuropathic ulcers, chronic skin ulcers. In particular, the wound healing gel of the present invention can be suitably used for wounds which do not show sufficient effect even after basic wound management (such as removal of necrotic tissue and the like, infection control, cleansing of the wound, and the like) has been performed and treatment has been carried out with wound dressings or topical agents for more than 4 weeks in principle.

From the perspective of achieving sufficient therapeutic effect, the wound healing gel of the present invention, although depending on the condition of the patient's wound, is preferably applied (adhered) to the wound site twice a week as a guide, with a total treatment duration capped at 8 weeks (maximum of 16 applications). The amount of wound healing gel used at one time (up to about 6 ml) can be adjusted as appropriate depending on the size of the wound.

### <Autologous platelet-rich plasma gel preparation kit for wound healing>

The present invention also comprises an autologous platelet-rich plasma gel preparation kit for wound healing, consisting of:
(I) blood collection needle,
(II) blood collection tube, and
(III) gelation reagent set,
wherein (III) gelation reagent set contains ascorbic acid solution at a concentration of 150 to 300 mg/mL, calcium chloride solution at a concentration of 1.0 to 3.0 wt. %, and thrombin in separate forms. Above wound healing gel of the present invention can be prepared by this kit.

As to (I) blood collection needle, any known one can be used without any limitation. (II) Blood collection tubes can be selected appropriately according to the amount of blood to be collected. Ascorbic acid solution at a concentration of 150 to 300 mg/mL, calcium chloride solution at a concentration of 1.0 to 3.0 wt. %, and thrombin contained in (III) gelation reagent set are same as those which are used in above method for producing a wound healing gel, and the descriptions of each item of above wound healing gel can be directly applied to the descriptions of each item of the reagent. Above thrombin is preferably in a freeze-dried form and is preferably stored frozen or refrigerated to prevent inactivation.

Hereinafter, an example of preparation procedure for wound healing gel using the autologous platelet-rich plasma gel preparation kit for wound healing is described. Furthermore, procedure for using the autologous platelet-rich plasma gel preparation kit for wound healing is not limited to the specific descriptions mentioned below.
(1) Use (I) blood collection needle to collect 4.5 mL of blood from the patients themselves or a third party into (II) blood collection tube in which 0.5 mL of an anticoagulant such as calcium chelator is pre-added to prevent blood coagulation.
(2) Mix well the blood with the anticoagulant and centrifuge at 3,000 to 4,500 ×*g* for 20 seconds to 1 minute to obtain platelet-rich plasma at a physiological platelet concentration (platelet concentration ratio: 1.0 times).
(3) Mix 2.5 mL of the platelet-rich plasma with ascorbic acid solution at a concentration of 150 to 300 mg/mL of an amount approximately equivalent to one-eighth of the amount of the plasma.
(4) To the mixed solution obtained in (3), add and mix a thrombin-dissolved calcium chloride solution at a concentration of 1.0 to 3.0 wt.% of equal amount to that of the ascorbic acid solution, and gelate to produce an autologous platelet-rich plasma gel for wound healing.

### EXAMPLES

Hereinafter, the present invention is described in detail with reference to examples and studies, but the present invention is not limited by the following examples and the like.

### 1. Production of a wound healing gel

Blood was collected in an amount of 5 mL from a patient with diabetic ulcers, and ACD-A solution (Biological Product Standard Blood Preservation Solution A) of an amount equivalent to one-ninth of the blood amount was mixed with the blood in a blood collection tube, which was then centrifuged at 4,236 ×*g* for 30 seconds to collect a plasma fraction containing platelets (PRP: platelet-rich plasma). The collected PRP was added in an amount of 2.5 mL into a 10 mL size tube. To the PRP, 313 µL of ascorbic acid solution at the concentration of 250 mg/mL was added and stirred. A calcium chloride-thrombin solution was prepared by dissolving 5,000 units of lyophilized thrombin per vial in 5 mL of 2% calcium chloride solution. To above ascorbic acid added PRP, 313 µL of calcium chloride-thrombin solution was added and stirred to induce gelation of the PRP and obtain the wound healing gel of the present invention.

### 2. Confirmation of wound healing effects of wound healing gel

An open-label, single-arm, multicenter study was conducted in 54 patients with diabetic ulcers to whom existing therapies did not respond (47 patients were the main target of efficacy analysis), and treatment with above wound healing gel was performed twice a week for up to 8 weeks. The percentage of cases in which the reduction rate of wound radius, as key evaluation criterion, was 50% or more (effective cases) was 80.9% (95% confidence interval [66.7, 90.9]), thus having confirmed the wound healing effect of the wound healing gel.

### 3. Comparison of the wound healing gel of the present invention with conventional products.

Blood was collected from the abdominal aorta of male rats (SD strain, 8 weeks old), and ACD-A solution (USP standard) of an amount equivalent to one-ninth of the blood amount was mixed with the blood in a blood collection tube, which was then centrifuged at 4,236 ×*g* for 30 seconds to collect a plasma fraction containing platelets (PRP: platelet-rich plasma). The collected PRP was added in an amount of 1.44 mL into a 2 mL size Eppendorf tube. To the PRP, 180 µL of ascorbic acid solution at the concentration of 250 mg/mL was added and stirred. A calcium chloride-thrombin solution was prepared by dissolving 5,000 units of lyophilized thrombin per vial in 5 mL of 2% calcium chloride solution. To above ascorbic acid added PRP, 180 µL of calcium chloride-thrombin solution was added and stirred to induce gelation of the PRP and obtain the wound healing gel of Example 1.

PRP collected in the same manner as above was added in an amount of 1.44 mL into a 2 mL size Eppendorf tube. To the PRP, 180 µL of ascorbic acid solution at the concentration of 500 mg/mL was added and stirred. A calcium chloride-thrombin solution was prepared by dissolving 5,000 units of lyophilized thrombin per vial in 5 mL of 10% calcium chloride solution. To above ascorbic acid added PRP, 180 µL of calcium chloride-thrombin solution was added and stirred to induce gelation of the PRP and prepare the wound healing gel of conventional products (Comparative example 1).

The hardness of the wound healing gels, Example 1 and Comparative Example 1, was measured using a grease degree meter (GP-DT type, Yoshida kagaku kikai Co., Ltd.). Specifically, Example 1 and Comparative example 1 of the wound healing gels were placed in respective containers and left to stand until the surface became flat. After adjusting the height of cones of specified dimensions and weight so that the tip of the cones touches the surface of the wound healing gels, the cones were dropped freely for one second, and the distances moved in the gel were measured. Fig. 1 shows the result.

As shown in Fig. 1, the wound healing gel of Example 1 was found to have a shorter distance moved within the gel compared to the wound healing gel of Comparative example 1. From this result, it was presumed that the wound healing gel of Example 1 is harder and more firmly gelled compared to the wound healing gel of Comparative example 1 (conventional products). Therefore, the wound healing gel of Example 1 can maintain its form for a longer time than conventional products when applied to skin wounds, can stay on the wound site for a long time, and can achieve sufficient therapeutic effects. Additionally, it has the advantage of being easy to handle because it is firmly gelled. Moreover, although the ascorbic acid concentration of the ascorbic acid solution and the calcium chloride concentration of the calcium chloride solution used in the preparation of the wound healing gel of the present invention are lower than those of conventional products, the obtained gel was unexpectedly harder than conventional products.

### INDUSTRIAL APPLICABILITY

The wound healing gel produced by the method of the present invention is firmly gelled compared to conventionally known wound healing gels using platelet-rich plasma, enabling to maintain its form for a long time when applied to skin wounds, sufficiently protecting the wound site and achieving a high therapeutic effect, also achieving sufficient effects on wounds and the like, which have not shown improvement trends with existing therapies. Additionally, it has the advantage of being easy to handle because it is firmly gelled.

## Claims

1. A method for producing a wound healing gel, comprising mixing:
(A) an effective amount of platelet-rich plasma,
(B) an ascorbic acid solution at a concentration of 150 to 300 mg/mL,
(C) a calcium chloride solution at a concentration of 1.0 to 3.0 wt.%, and
(D) thrombin,
wherein
a mixing ratio between (A) platelet-rich plasma and (B) ascorbic acid solution is 1.0 to 3.0 mL of (B) ascorbic acid solution per 8.0 mL of (A) platelet-rich plasma,
a mixing ratio between (A) platelet-rich plasma and (C) calcium chloride solution is 1.0 to 3.0 mL of (C) calcium chloride solution per 8.0 mL of (A) platelet-rich plasma, and
an amount of (D) thrombin is 500 to 2,000 U per 8.0 mL of (A) platelet-rich plasma.

2. The method for producing a wound healing gel according to claim 1, wherein (D) thrombin being dissolved in (C) calcium chloride solution is mixed with (A) platelet-rich plasma and (B) ascorbic acid solution.

3. The method for producing a wound healing gel according to claim 2, wherein with (A) platelet-rich plasma, (B) ascorbic acid solution is mixed, and then the calcium chloride solution of thrombin is mixed.

4. The method for producing a wound healing gel according to claim 1, further comprising obtaining (A) platelet-rich plasma by centrifuging blood to separate platelet concentrates from other blood components.

5. The method for producing a wound healing gel according to claim 4, wherein the blood is treated with a calcium chelator.

6. The method for producing a wound healing gel according to claim 4, wherein the conditions of the centrifugation is 3,000 to 4,500 ×*g* for 20 seconds to 1 minute.

7. A method for producing a wound healing gel sheet, comprising mixing:
(A) an effective amount of platelet-rich plasma,
(B) an ascorbic acid solution at a concentration of 150 to 300 mg/mL,
(C) a calcium chloride solution at a concentration of 1.0 to 3.0 wt.%, and
(D) thrombin,
wherein
a mixing ratio between (A) platelet-rich plasma and (B) ascorbic acid solution is 1.0 to 3.0 mL of (B) ascorbic acid solution per 8.0 mL of (A) platelet-rich plasma,
a mixing ratio between (A) platelet-rich plasma and (C) calcium chloride solution is 1.0 to 3.0 mL of (C) calcium chloride solution per 8.0 mL of (A) platelet-rich plasma, and
an amount of (D) thrombin is 500 to 2,000 U per 8.0 mL of (A) platelet-rich plasma.

8. A wound healing gel obtained by mixing:
(A) an effective amount of platelet-rich plasma,
(B) an ascorbic acid solution at a concentration of 150 to 300 mg/mL,
(C) a calcium chloride solution at a concentration of 1.0 to 3.0 wt.%, and
(D) thrombin,
wherein
a mixing ratio between (A) platelet-rich plasma and (B) ascorbic acid solution is 1.0 to 3.0 mL of (B) ascorbic acid solution per 8.0 mL of (A) platelet-rich plasma, and
a mixing ratio between (A) platelet-rich plasma and (C) calcium chloride solution is 1.0 to 3.0 mL of (C) calcium chloride solution per 8.0 mL of (A) platelet-rich plasma.

9. The wound healing gel according to claim 8, wherein (D) thrombin being dissolved in (C) calcium chloride solution is mixed with (A) platelet-rich plasma and (B) ascorbic acid solution.

10. The wound healing gel according to claim 9, wherein the wound healing gel is obtained by mixing with (A) platelet-rich plasma, (B) ascorbic acid solution, and then the calcium chloride solution of thrombin.

11. An autologous platelet-rich plasma gel preparation kit for wound healing, consisting of:
(I) blood collection needle,
(II) blood collection tube, and
(III) gelation reagent set,
wherein (III) gelation reagent set contains ascorbic acid solution at a concentration of 150 to 300 mg/mL, calcium chloride solution at a concentration of 1.0 to 3.0 wt.%, and thrombin in separate forms.
